# EUROPEAN PATENT APPLICATION

(11) **EP 4 826 359 A1**
(43) Date of publication of application: **21.10.2026**
(21) Application number: 26171998.3
(22) Date of filing: 13.04.2026
(51) Int. Cl.: G01N 21/47, C12M 1/34, G01N 21/45, G01N 21/25, G01N 21/41, G01N 21/84

(54) **LABEL-FREE QUANTIFICATION SYSTEM AND METHOD FOR SECRETED PROTEINS USING OPTICAL IMAGING**

(30) Priority: 14.04.2025 KR 20250048170; 20.03.2026 KR 20260050538
(71) Applicant: Korea Advanced Institute of Science and Technology, Daejeon 34141 (KR); Tomocube, Inc., Daejeon 34127 (KR)
(72) Inventor: PARK, YongKeun, 34141 Daejeon (KR); PARK, Wei Sun, 34141 Daejeon (KR)
(74) Representative: Keller Schneider Patent- und Markenanwälte AG

(57) **Abstract**

Disclosed is a label-free quantitative phase imaging-based secreted protein quantification system and method. A secreted protein quantification method for quantifying the concentration of secreted proteins in the cell culture medium using quantitative phase imaging may include acquiring three-dimensional (3D) data that represents the optical property of the culture medium in which a cell is cultured using a quantitative phase imaging device, calculating the refractive index difference between a reference element contained in the culture medium and the culture medium, and converting the calculated refractive index difference to the concentration of secreted proteins based on a refractive index increase factor of protein.

## Description

### BACKGROUND

### 1. Field of the Invention

The present disclosure relates to label-free quantification technology for the concentration of proteins secreted in the cell culture medium, including optical imaging technology that uses optical phase, amplitude, scattering, or refractive index information, and more particularly, to a secreted protein quantification system and method for computing the concentration and secretion rate of secreted proteins in real time based on the three-dimensional (3D) refractive index distribution of the culture medium.

### 2. Description of the Related Art

In a cell culture-based biopharmaceutical production process, it is crucial to accurately quantify the concentration of secreted substances, such as antibodies or other proteins. In particular, since the concentration and the production speed of secreted proteins during a culture process directly affect the productivity evaluation of cell line, process optimization, and quality management, technology capable of precisely measuring the same is required.

In the conventional art, enzyme immunoassay (ELISA), protein A affinity chromatography or various biosensor-based analysis methods have been widely used to quantify the protein concentration in the culture medium. However, since most of these methods involve collecting samples from the culture medium and then performing offline analysis, it is difficult to continuously monitor the change in concentration in real time and there is a latency in the analysis results. Also, repeatedly collecting samples may affect a culture environment, or may increase the risk of contamination.

Meanwhile, methods using fluorescent proteins or reporter genes have also been proposed. However, since foreign genes need to be introduced to cells, this may burden the cells' metabolism and has limitations in that the concentration of secreted proteins may not be directly reflected. Also, since the expression and maturation of fluorescent signals require a certain amount of time, there are limitations in tracking the dynamic change with high temporal resolution.

Also, while techniques for estimating the concentration of substances in a solution using refractive index or optical property are known, the conventional methods are mainly limited to measurement based on a single point or surface reaction, or have an issue of failing to sufficiently reflecting spatially distributed information. In particular, there are limitations in detecting the minute change in concentration occurring in a complex environment, such as cell culture medium, with high sensitivity and quantitatively analyzing the same over time.

Further, the conventional techniques are often difficult to apply to high-throughput environments that involve the simultaneous comparative analysis of multiple cell lines or secretion characteristics at a single cell level, and there are technical limitations in being directly utilized for in-line monitoring during the process.

Therefore, there is a continuously growing need for new technology that may precisely quantify the concentration of secreted proteins in the culture medium in real time and may continuously track the change over time, while minimizing impact on a cell culture process.

### SUMMARY

An example embodiment provides a label-free quantification system and method for secreted proteins using optical imaging.

According to at least one example embodiment, there is provided a method of quantifying the concentration of secreted proteins in the cell culture medium using optical imaging, the method including acquiring three-dimensional (3D) data that represents the optical property of the culture medium in which a cell is cultured; computing the change in refractive index of the culture medium from the 3D data; and converting the change in refractive index to the concentration of secreted proteins based on the relationship with the protein concentration.

According to an aspect, the computing of the change in refractive index may include computing the relative or absolute change in refractive index by comparing the refractive index of the culture medium with a reference value.

According to another aspect, the reference value may be determined based on at least one of a reference element, initial time point data, control group data, and external calibration data.

According to still another aspect, the change in refractive index may be calculated based on the difference between a refractive index value as the reference value and a representative refractive index value of a spatially defined area of the culture medium.

According to still another aspect, the secreted protein quantification method may further include computing the rate of change in concentration of secreted proteins per unit time by comparing 3D data acquired at two different time points.

According to still another aspect, the rate of change in concertation per unit time may be computed by spatially integrating the local refractive index distribution around a single cell.

According to still another aspect, the refractive index of the culture medium may be calculated after calibrating the refractive index drift according to the temperature change.

According to still another aspect, a culture environment of the culture medium may be maintained in the temperature range of ±0.1°C.

According to still another aspect, the secreted protein quantification method may further include calibrating the change in background refractive index using refractive index data of the culture medium of control cells that do not produce antibodies.

According to still another aspect, the secreted protein quantification method may further include comparing the concentration of secreted proteins computed for each of a plurality of culture chambers.

According to still another aspect, the secreted protein quantification method may further include selecting a high-productivity cell line based on the comparison result.

According to still another aspect, the secreted protein quantification method may further include generating a control signal when the concentration or secretion rate of secreted proteins exceeds a set threshold.

According to still another aspect, a culture vessel in which the cell is cultured may include a multi-well plate or a microfluidic device.

According to at least one example embodiment, there is provided a computer program stored in a non-transitory computer-readable recording medium to execute the method on a computer device in conjunction with the computer device.

According to at least one example embodiment, there is provided a non-transitory computer-readable recording medium storing a computer program to execute the method on a computer device.

According to at least one example embodiment, there is provided a secreted protein quantification system for quantifying the concentration of secreted proteins in the cell culture medium using optical imaging, the secreted protein quantification system including a culture vessel in which a cell is cultured; an optical imaging device configured to acquire 3D data that represents the optical property of the culture medium contained in the culture vessel; and a data processing unit configured to compute the change in refractive index of the culture medium from the 3D data, and to convert the change in refractive index to the concentration of secreted proteins based on the relationship with the protein concentration.

According to an aspect, the data processing unit may be configured to compute the rate of change in concentration per unit time by comparing the 3D data acquired at different time points, and the secreted protein quantification system may further include an output unit configured to output the concentration or secretion rate of secreted proteins; and a process control unit configured to generate a control signal based on the concentration or secretion rate.

According to some example embodiments, since proteins may be quantified in a label-free and non-destructive manner, it is possible to minimize stress applied to cells.

Also, since in-line monitoring is possible, it is possible to continuously track a protein production status during a culture process and to utilize the same for process control.

Also, it is possible to precisely quantify the change in concentration down to the level of tens of micrograms/milliliter based on the refractive index sensitivity of the level of 1×10⁻⁵.

Also, since secretion rate analysis at the single-cell level is possible, it is possible to precisely evaluate the difference in productivity between cells.

Also, since high-productivity cell lines may be quickly selected, it is possible to reduce a bioprocess development period and to improve the production efficiency.

### BRIEF DESCRIPTION OF THE DRAWINGS

These and/or other aspects, features, and advantages of the invention will become apparent and more readily appreciated from the following description of embodiments, taken in conjunction with the accompanying drawings of which:
FIG. 1 is a diagram illustrating a configuration of a label-free secreted protein quantification system based on quantitative phase imaging according to an example embodiment;
FIG. 2 is a flowchart illustrating an example of a secreted protein quantification method according to an example embodiment;
FIG. 3 is a block diagram illustrating an example of a computer device according to an example embodiment;
FIG. 4 illustrates an example of a three-dimensional (3D) refractive index distribution image acquired using a quantitative phase imaging device according to an example embodiment; and
FIG. 5 is a graph showing the change in refractive index difference (ΔRI) according to IgG concentration according to an example embodiment.

### DETAILED DESCRIPTION

Hereinafter, example embodiments will be described with reference to the accompanying drawings.

The example embodiments relate to a label-free optical imaging-based secreted protein quantification system and method for quantifying the concentration of antibodies or other proteins secreted in the cell culture medium in real time using optical imaging technology.

As used herein, the term "optical imaging" may comprehensively refer to technology for acquiring the physical or optical property of a target using light and reconstructing the same in the form of an image or data. The optical imaging may include not only simple intensity-based imaging, but also various methods for quantitatively or qualitatively measuring the optical prosperity of the target based on phase, interference, diffraction, scattering, polarization, or combination thereof.

According to an example embodiment, the optical imaging may include technology capable of estimating the refractive index distribution of the culture medium or the change therein, for example, quantitative phase imaging (QPI) technology. The quantitative phase imaging may include various implementation methods, such as digital holography, interferometric microscopy, Fourier ptychographic microscopy, phase retrieval imaging, and optical diffraction tomography.

Also, the optical imaging may include not only a method of directly measuring phase information, but also an indirect method of reconstructing phase or refractive index information using interference patterns, diffraction patterns, or image data acquired from multiple angles. For example, the optical imaging may include technology for reconstructing the 3D optical property of a target using an interferometric image, a multi-focus image, a multi-angle image, and a time-series image.

Also, the optical imaging may include a method of using single-wavelength or multi-wavelength light, a method of using coherent light or incoherent light, and a measurement method based on transmission, reflection, or scattering, and accordingly may be implemented by various optical systems and configurations (e.g., microscope system, holographic system, imaging sensor-based system).

Therefore, optical imaging set forth herein is not limited to a specific implementation method, and should be understood to include all light-based measurement and imaging technologies that may estimate the refractive index of the culture medium and the change therein.

The example embodiments may acquire the 3D refractive index distribution of the culture medium or optical property data corresponding thereto, and may compute the concentration of secreted proteins from the optical property. According to an example embodiment, the protein concentration may be quantified using the optical difference between a reference element with the known refractive index and the culture medium.

In an example embodiment, the protein concentration may be computed using the protein's refractive index increase factor (dn/dc). Here, dn/dc may represent the refractive index increase factor indicating the ratio of the change in refractive index (dn) to the change in concentration (dc). However, the present invention is not limited to this linear relationship.

According to another example embodiment, the relationship between the optical property and the protein concentration may be defined using a calibration curve based on calibration data that is acquired in advance, and the calibration curve may be expressed in the form of a linear or nonlinear function.

Also, the relationship between the optical property and the protein concentration may be expressed by a nonlinear model that is configured using a polynomial, an exponential function, a logarithmic function, or combination thereof, and a different model may be applied depending on a specific protein type, a culture condition, or a measurement environment.

According to another example embodiment, the relationship between the optical property and the protein concentration may be defined by a mapping function that is learned by a machine learning or deep learning-based model. For example, the protein concentration may be estimated from the optical property using a regression model, a neural network, a kernel-based model, or the combination thereof

Also, computation of the protein concentration may be performed based on multivariate analysis that simultaneously considers not only the optical property at a single time point but also the change over time, spatial distribution patterns, or a plurality of optical parameters.

Therefore, herein, computation of the protein concentration is not limited to simple linear conversion based on dn/dc, and should be understood to include all various mathematical models, empirical models, or data-based learning models that may define the relationship between the optical property and the protein concentration.

The secreted protein quantification system according to an example embodiment is configured to detect the refractive index change of 1×10⁻⁴ or less, enabling the quantification of the secretion rate per unit time (dC/dt). Also, the secreted protein quantification system may improve the quantification accuracy including temperature drift calibration and control group-based calibration, and may be utilized for selection of high-productivity cell lines and process control in a multi-well or a microfluidic platform.

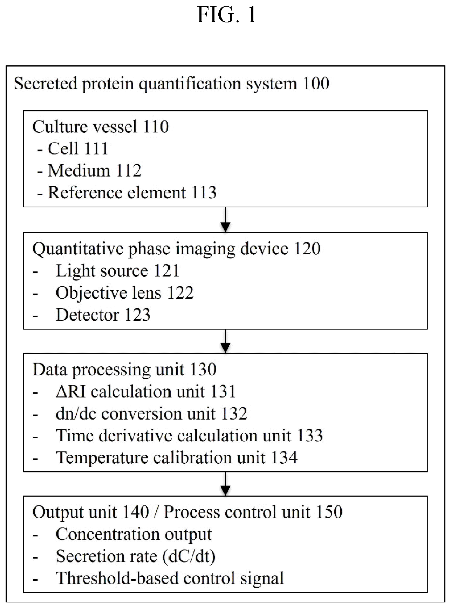

FIG. 1 is a diagram illustrating a configuration of a label-free secreted protein quantification system based on quantitative phase imaging according to an example embodiment.

Referring to FIG. 1, a secreted protein quantification system 100 according to an example embodiment may include a culture vessel 110, a quantitative phase imaging device 120, a data processing unit 130, an output unit 140, and a process control unit 150.

The culture vessel 110 contains a cell 111 and a medium 112, and the cell 111 may secrete antibodies or other proteins into the medium 112. Also, the culture vessel 110 may contain a reference element 113 with a known refractive index. The reference element 113 is to provide a refractive index reference value, and may be implemented in the form of a fixed structure that is formed inside the microsphere or the culture vessel 110.

The quantitative phase imaging device 120 may include a light source 121, an objective lens 122, and a detector 123. The light source 121 may irradiate light onto the culture vessel 110, the objective lens 122 may collect the light that passes through or reflected from the culture vessel 110, and the detector 123 may acquire an image that includes phase information of the light. The quantitative phase imaging device 120 may reconstruct the three-dimensional (3D) refractive index distribution of the medium 112 using the image acquired from the detector 123.

The data processing unit 130 may compute the concentration of secreted proteins based on refractive index distribution data that is provided from the quantitative phase imaging device 120, and may include a ΔRI calculation unit 131, a dn/dc conversion unit 132, a time derivative calculation unit 133, and a temperature calibration unit 134. The ΔRI calculation unit 131 may calculate the refractive index difference between the reference element 113 and the medium 112, and the dn/dc conversion unit 132 may convert the refractive index difference to the protein concentration based on a refractive index increase factor (dn/dc) of proteins. Also, the time derivative calculation unit 133 may compare refractive index distributions acquired at different time points, and may compute the rate of change in concentration per unit time (dC/dt), and the temperature calibration unit 134 may calibrate the refractive index drift according to the temperature change.

The output unit 140 may output the concentration or secretion rate (dC/dt) of secreted proteins computed by the data processing unit 130, and may provide the same to a user or may store the same.

The process control unit 150 may generate a control signal based on information that is provided from the output unit 140 or the data processing unit 130. The control signal may be generated when the concentration or the secretion rate of secreted proteins exceeds a preset threshold, and may be used to control a culture process.

Meanwhile, the data processing unit 130, the output unit 140, and the process control unit 150 may be implemented by a computer device that includes at least one processor, and may perform the aforementioned function by executing a program instruction stored in a memory.

Although FIG. 1 describes an example embodiment of acquiring the 3D refractive index distribution of the culture medium using the quantitative phase imaging device 120, it is only an example provided to help understanding of the present invention.

That is, an optical imaging device used herein is not limited to a specific quantitative phase imaging method, and may include a variety of optical imaging technology that may acquire or estimate the refractive index of the culture medium or the optical property corresponding thereto.

For example, the optical imaging device may be implemented as various imaging devices based on digital holography, interferometric microscopy, Fourier ptychographic microscopy, optical diffraction tomography, phase retrieval imaging, or any optical principle similar thereto, and may also include not only a method of directly measuring phase information but also a method of indirectly reconstructing phase information.

Therefore, the quantitative phase imaging device 120 illustrated in FIG. 1 corresponds to a single specific implementation example of the optical imaging device, and the scope of the present invention is not limited thereto and should be understood to include a case in which various optical imaging techniques are used.

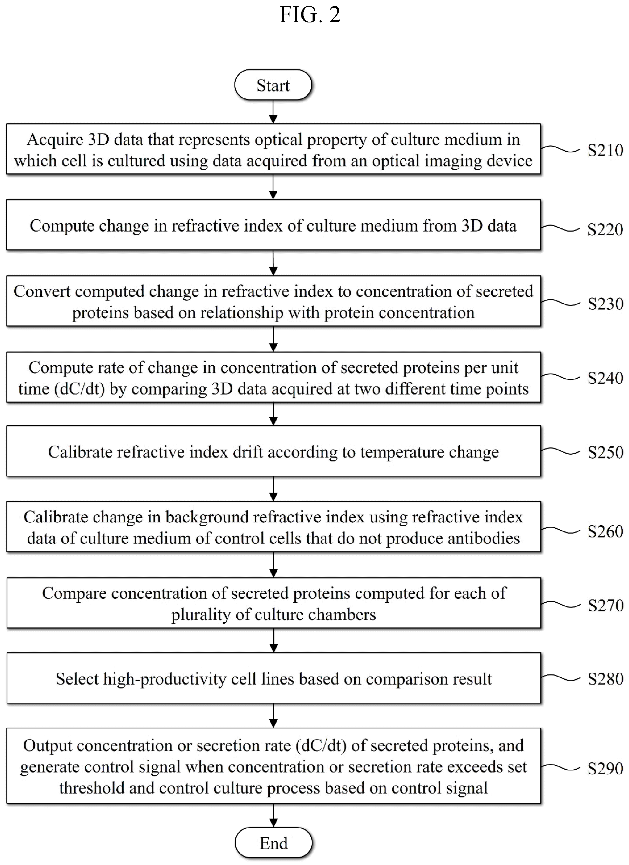

FIG. 2 is a flowchart illustrating an example of a secreted protein quantification method according to an example embodiment. The secreted protein quantification method according to the example embodiment may be performed by a computer device that includes the data processing unit 130, the output unit 140, and the process control unit 150 described above as logical components.

In operation S210, the computer device may acquire 3D data that represents the optical property of the culture medium in which a cell is cultured using data acquired from an optical imaging device through the data processing unit 130. Here, the 3D data may include the refractive index distribution of the culture medium or the optical property corresponding thereto, and may be reconstructed based on an interference image, a phase image, a diffraction pattern, or a multi-angle image. Also, the 3D data may be generated using a phase retrieval algorithm, a tomography-based reconstruction algorithm, or a data-based estimation algorithm.

In operation S220, the computer device may compute the change in refractive index of the culture medium from the 3D data through the data processing unit 130. According to an example embodiment, the change in refractive index may be computed as the relative or absolute change by comparing the refractive index of the culture medium with a reference value. Here, the reference value may be determined based on at least one of a reference element, a refractive index value at an initial time point, control group data, and external calibration data.

Also, the change in refractive index may be calculated based on the difference between a refractive index value as the reference value and a representative refractive index value of a spatially defined area within the culture medium. Here, the data processing unit 130 may define a region of interest (ROI) using image segmentation, threshold-based classification, or object recognition techniques, and a representative refractive index value for the region of interest may be computed by various statistical methods including the mean, median, weighted mean, or combination thereof. Also, the weighted mean may be computed by considering a spatial location, signal strength, reliability, or noise characteristic.

In operation S230, the computer device may convert the computed change in refractive index to the concentration of secreted proteins based on the relationship with the protein concentration through the data processing unit 130. In this regard, the conversion may be performed based on various models that define the relationship between the change in refractive index and the protein concentration. For example, a refractive index increase factor(dn/dc)-based linear model, a pretrained calibration curve, a nonlinear function model, or a machine learning-based mapping model may be applied.

In operation S240, the computer device may compute the rate of change in concentration of secreted proteins per unit time (dC/dt) by comparing 3D data acquired at two different time points through the data processing unit 130. In an example embodiment, the rate of change in concentration may be computed by spatially integrating the local refractive index distribution around a single cell. Here, the data processing unit 130 may perform registration between temporally continuous data to track the change at the same location, and may precisely calculate the secretion rate by setting a region of interest and by integrating the change in refractive index within the set region of interest.

In operation S250, the computer device may calibrate the refractive index drift according to the temperature change through the data processing unit 130. Here, the cell culture environment may be maintained within the temperature range of ±0.1°C. In this regard, the data processing unit 130 may remove a drift component by applying a calibration factor that reflects the temperature dependence of the refractive index using temperature information acquired from a temperature sensor or by analyzing the trend of change in refractive index over time.

In operation S260, the computer device may calibrate the change in background refractive index using refractive index data of the culture medium of control cells that do not produce antibodies using the data processing unit 130. Here, the data processing unit 130 may extract only a signal corresponding to actual protein secretion by removing a non-specific change component using the change in refractive index acquired from a control group as a reference signal.

In operation S270, the computer device may compare the concentration of secreted proteins computed for each of a plurality of culture chambers through the data processing unit 130. In this regard, the data processing unit 130 may process data acquired from a multi-well or a multi-channel in parallel, and may evaluate relative productivity by normalizing and comparing a concentration value for each chamber.

In operation S280, the computer device may select high-productivity cell lines based on the comparison result through the data processing unit 130. Here, the data processing unit 130 may automatically identify a cell or cell population with high productivity based on a preset threshold or a rank-based criterion.

In operation S290, the computer device may output the concentration or secretion rate (dC/dt) of secreted proteins through the output unit 140, and may generate a control signal when the concentration or the secretion rate exceeds a set threshold and may control a culture process based on the control signal through the process control unit 150. Here, the output unit 140 may provide graph, numerical data, or visualization information, and the process control unit 150 may automatically adjust culture conditions (e.g., temperature, medium replacement, flow rate), or may generate a warning signal.

Meanwhile, the culture vessel in which the cells are cultured may be implemented in the form of a multi-well plate or a microfluidic device.

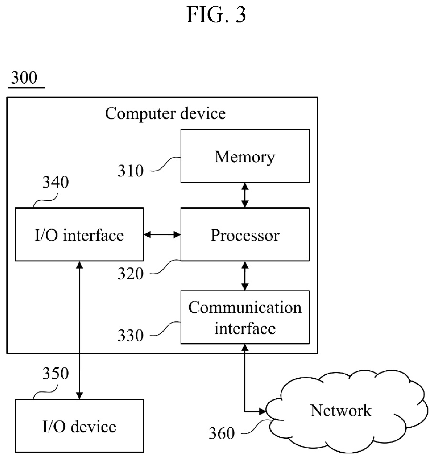

FIG. 3 is a block diagram illustrating an example of a computer device according to an example embodiment. For example, a computer device 300 may correspond to the computer device that implements the data processing unit 130, the output unit 140, and the process control unit 150 described above. Referring to FIG. 3, the computer device 300 may include a memory 310, a processor 320, a communication interface 330, and an input/output (I/O) interface 340.

The memory 310 may include a permanent mass storage device, such as random access memory (RAM), read only memory (ROM), and disk drive, as a computer-readable recording medium. Here, the permanent mass storage device such as ROM and disk drive may be included in the computer device 30 as a separate permanent storage device distinct from the memory 310. Also, the memory 310 may store an operating system (OS) and at least one program code.

Such software components may be loaded from another computer-readable recording medium separate from the memory 310 to the memory 310. Such separate computer-readable recording medium may include a computer-readable recording medium, such as a floppy drive, a disc, a tape, a DVD/CD-ROM drive, and a memory card. In another example embodiment, the software components may be loaded to the memory 310 through the communication interface 330 instead of the computer-readable recording medium. For example, the software components may be loaded to the memory 310 of the computer device 300 based on a computer program installed by files received through a network 360.

The processor 320 may be configured to process instructions of the computer program by performing basic arithmetic, logical, and I/O operations. The instructions may be provided from the memory 310 or the communication interface 330 to the processor 320. For example, the processor 320 may be configured to execute the received instructions according to a program code stored in a storage device, such as the memory 310.

The communication interface 330 may provide a function for the computer device 300 to communicate with another device over the network 360. For example, a request or an instruction created by the processor 320 of the computer device 300 according to a program code stored in the storage device such as the memory 310, data, a file, and the like may be transmitted to other devices through the network 360 under control of the communication interface 330. Inversely, a signal or an instruction from another device, data, a file, and the like may be received by the computer device 300 through the network 360 and the communication interface 330 of the computer device 300.

The I/O interface 340 may be a method for interfacing with an I/O device 350. For example, an input device may include a device, such as a microphone, a keyboard, and a mouse, and an output device may include a device, such as a display and a speaker. As another example, the I/O interface 340 may be a method for interacting with a device in which functions for input and output are integrated into one, such as a touchscreen. The I/O device 350 may be configured as a single device with the computer device 300.

Also, in other example embodiments, the computer device 300 may include the number of components greater than or less than the number of components shown in FIG. 3. However, there is no need to clearly illustrate the most conventional components. For example, the computer device 300 may be implemented to include at least a portion of the I/O device 350, or may further include other components, such as a transceiver and a database.

An example embodiment may quantify the concentration of proteins secreted in the cell culture medium without labeling or sampling.

Also, an example embodiment may quantitatively calculate the concentration of secreted proteins based on the physical principle, using the 3D refractive index distribution of the culture medium.

Also, an example embodiment may provide high-precision quantification technology that may resolve the change in protein concentration at the level of tens of micrograms/milliliter by detecting the minute change in refractive index of 1×10⁻⁴ or less.

Also, an example embodiment may provide a method that may qualitatively evaluate the productivity kinetics of a cell or cell population by computing the secretion rate per unit time (dC/dt).

Also, an example embodiment may provide a system that may be utilized to control a high-productivity cell line screening and culture process in a multi-well or microfluidic platform environment.

### (1) Basic principle

The present invention is based on the principle of acquiring the 3D refractive index distribution of the cell culture medium using a quantitative phase imaging device. When a cell secretes antibodies or other proteins into the culture medium, the refractive index of the culture medium increases and this change in refractive index has the characteristic of being proportional to the change in protein concentration.

Here, the relationship between the change in refractive index(Δn) and the change in protein concentration (Δc) may be expressed as follows. $Δc = Δn / \left(dn/dc\right)$

Here, dn/dc represents the refractive index increase factor of protein, and denotes the ratio of the change in refractive index according to the change in protein concentration. For example, in the case of antibodies, dn/dc may have a value of about 0.185 mL/g.

### (2) Reference refractive index reference element

For accurate protein concentration calculation, the present invention may include a reference element with a known refractive index in the culture medium.

The reference element is to provide a refractive index reference value, and may be implemented, for example, as a microsphere bead with a known refractive index or a fixed structure formed inside the culture vessel.

By calculating the refractive index difference between the reference element and the medium, the optical system drift or system error may be calibrated and accordingly, the concentration may be more accurately computed.

### (3) High sensitivity implementation

The present invention may be configured to detect the valid change in refractive index of 1×10⁻⁴ or less by applying spatial averaging, temporal averaging, and reference-based differential referencing to 3D refractive index distribution data.

When the refractive index increase factor (dn/dc) of the protein is assumed as about 0.185 mL/g, the minimum concentration change detectable at the sensitivity may be calculated as follows. $Δc \approx Δn / \left(dn/dc\right) \approx \left(1\times {10}^{- 4}\right) / 0.185 \approx 0.54 mg / mL$

Therefore, according to the present inventio, it is possible to distinguish the change in protein concentration up to the level of about 500 µg/mL.

Also, when it is possible to detect a smaller Δn value due to improvement in the sensitivity of the 3D refractive index measurement device, the minimum concentration change detectable according to the relationship also proportionally decreases, which makes it possible to detect the even minute change in concentration.

### (4) Calculation of secretion rate based on time difference.

The present invention may compute the change in concentration per unit time (dC/dt) by calculating the difference between 3D refractive index distributions acquired at different time points.

Through this, it is possible to quantify the amount of proteins secreted by a cell or cell population per unit time, and to precisely evaluate the productivity or secretion kinetics of a cell line.

### (5) Temperature calibration

Since the refractive index of water and culture medium has the characteristic of varying with temperature, the present invention may include a function for calibrating the temperature drift.

In an example embodiment, the culture environment may be maintained within the range of ±0.1 °C or less, and the fluctuation in the refractive index due to the temperature change may be algorithmically calibrated. This may minimize measurement errors caused due to the change in an external environment.

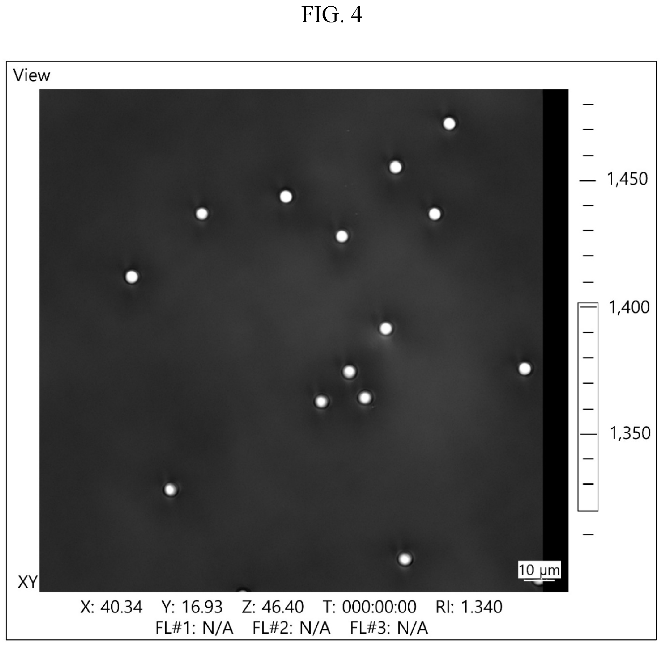

FIG. 4 illustrates an example of a 3D refractive index distribution image acquired using a quantitative phase imaging device according to an example embodiment.

Referring to FIG. 4, the 3D refractive index distribution image acquired through the quantitative phase imaging device may be illustrated in a state in which microsphere beads with known refractive indices are dispersed in IgG solutions of different concentrations. The image may be the result of reconstructing the refractive index distribution for the 3D space of x, y, and z, and a refractive index value at each location may be visually represented.

In the image, a brightly displayed area corresponds to a microsphere bead, while a relatively darkly displayed area may correspond to the medium. The microsphere bead functions as a reference element with a known refractive index, and the distribution of difference between the refractive index of microsphere bead (RI_bead) and the refractive index of surrounding medium (RI_medium), that is, ΔRI = RI_bead - RI_medium may appear in the image.

According to an example embodiment, as the IgG concentration increases, the refractive index of the medium (RI_medium) increases and accordingly, the ΔRI value may show a tendency to decrease. In particular, the ΔRI value may have the characteristic of substantially linearly varying with respect to the IgG concentration. Through this, it may be confirmed that the protein concentration may be quantitatively computed based on the refractive index difference.

Therefore, as illustrated in FIG. 4, by utilizing the refractive index difference between the microsphere bead as the reference element and the medium, the concentration of secreted proteins may be precisely computed while minimizing the influence of system error or external environmental change.

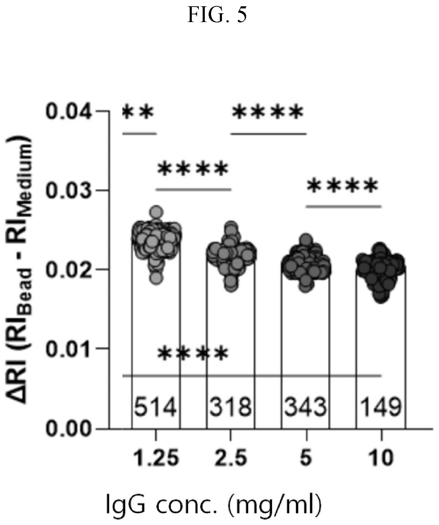

FIG. 5 is a graph showing the change in refractive index difference (ΔRI) according to IgG concentration according to an example embodiment.

Referring to FIG. 5, the x-axis may represent the IgG concentration (mg/mL), and the y-axis may represent the refractive index difference between the microsphere bead and the medium, which is measured by including the microsphere bead with the known refractive index in the IgG solution, that is, ΔRI = RI_bead - RI_medium.

In the graph, each datapoint may represent the result that is iteratively measured under the same condition, and the average value and the distribution of ΔRI values under the respective concentration conditions may be illustrated together.

According to an example embodiment, the refractive index (RI_medium) of the medium increases according to the increase in the IgG concentration, while the refractive index of the microsphere bead (RI_bead) is maintained consistent. Therefore, the ΔRI value may show tendency to decrease according to the IgG concentration. In particular, the ΔRI value may have the characteristic of substantially linearly decreasing with respect to the increase in IgG concentration.

It can be verified that this linear relationship matches the theoretical equation based on the refractive index increase factor (dn/dc) of the protein, which supports that it is possible to quantitatively compute the protein concentration using the ΔRI value.

Also, as illustrated in FIG. 5, since the difference in ΔRI value between different concentration conditions may be meaningfully distinguished in the statistical aspect, it can be verified that the method according to the present invention enables quantification with high resolution even for the minute change in concentration.

Hereinafter, various example embodiments will be described in detail to help the understanding of the present invention. However, the example embodiments described below are not to limit the technical spirit of the present invention, and are simply examples for one of ordinary skill in the art to easily carry out the present invention. Therefore, the scope of the present invention is not limited to the following example embodiments, and should be defined by the matters described in the claims and the equivalents thereof.

Example embodiments may be configured to measure the amount of antibodies secreted within the cell culture medium in real time without fluorescent labeling or separate sample collection. Also, the example embodiments may be implemented to continuously monitor the antibody concentration during a culture process while minimizing the impact on the cell growth and metabolism.

Example embodiments may be configured to select high-productivity cell lines based on the change in antibody concentration during a culture process, or to perform automated quality control of the culture process. Also, the example embodiments may quantify the antibody concentration based on high accuracy and reproducibility, and may be applied to evaluate the secretion rate at the single-cell level.

Example embodiments may be utilized to improve the production efficiency in a biopharmaceutical production process, to shorten a process development period, and to reduce the workload of experimenters.

Example embodiments may be configured to acquire a 3D phase-based image for cells and the culture medium using label-free quantitative phase imaging technology, and through this, to precisely measure the change in refractive index (RI) of the culture medium. As cells secrete antibodies or proteins into the culture medium, the refractive index of the culture medium may increase, and this change in refractive index may be utilized as a physical indicator corresponding to the protein concentration.

Example embodiments may be configured to include a refractive index reference element that is a basis within the culture medium, thereby calibrating the external environmental change or measurement deviation and computing an absolute concentration value. The reference element may be implemented to include a microsphere bead with a known refractive index, or to include a fixed structured formed within the culture vessel.

Example embodiments may be configured to calculate the refractive index difference between the reference element and the culture medium from the 3D refractive index distribution that is acquired through quantitative phase imaging, and to convert the same into the antibody concentration based on the refractive index increase factor (dn/dc) of protein. Here, the refractive index increase factor may represent the rate of change in refractive index according to the change in unit concentration, and may have a value of about 0.185 mL/g in the case of antibodies.

Also, example embodiments may be configured to calculate the change in concentration over time by comparing 3D refractive index distributions acquired at different time points and to compute the secretion rate per unit time based on this. Through this, the production characteristic of a single cell or cell population may be quantitatively evaluated.

In addition, example embodiments may be configured to include a calibration technique using a control group. By measuring the change in refractive index while culturing a control cell line that does not produce antibodies, under the same conditions, and by computing the change in background refractive index based on the measured change in refractive index, it is possible to distinguish the non-specific change in refractive index and the change caused by antibodies. Through this, quantification accuracy and measurement reliability may be improved.

In summary, the example embodiments may be configured to quantify the antibody concentration within the culture medium in real time in a label-free manner using the 3D refractive index distribution and the reference element, and to perform integrated monitoring that includes analysis of change over time and control group calibration.

### [Embodiment 1: Method of quantifying antibody concentration using microsphere]

In this example embodiment, a method of quantifying the antibody concentration within the culture medium by utilizing a microsphere with a prespecified refractive index as a reference element is described.

Initially, a cell line that produces antibodies may be inoculated into a culture vessel (e.g., one well of multi-well plate) under appropriate culture conditions, and a polystyrene microsphere with a diameter of several micrometers (e.g., 5 to 10 µm) may be added into the culture medium. The refractive index of the polystyrene microsphere is known to be about 1.59 (based on λ=589 nm), and the initial refractive index of the culture medium may be about 1.334.

By acquiring the 3D refractive index distribution at an initial time point (t0) using a quantitative phase imaging device, the refractive index of the medium around the microsphere may be precisely measured. Subsequently, as a cell is cultured and the antibody proteins are secreted into the medium, the refractive index of the culture medium may increase and, at a subsequent time point (t1), the refractive index of the medium may be measured to be in an elevated state.

For example, when the antibody concentration increases by 1 mg/mL, the refractive index of the medium may increase by about 0.000185 according to the refractive index increase factor (dn/dc ≈ 0.185 mL/g) of protein. This minute change may appear as the change in the refractive index distribution in a boundary area between the microsphere and the medium, and may be detected through quantitative phase imaging data.

A system according to the present invention may compute the refractive index of the medium by extracting refractive index data at the voxel level around the microsphere and by comparing the same with the known refractive index of the microsphere. Subsequently, the refractive index difference between time points may be calculated, and may be divided by the refractive index increase factor of protein, and the result may be converted into the change in concentration.

Specifically, the change in concentration may be expressed as follows. $ΔC = \left(RI\left(t1\right)-RI\left(t0\right)\right) / \left(dn/dc\right)$

Here, ΔC denotes the change in protein concentration per unit volume.

Also, the system may quantify the secretion rate by computing the rate of change in concentration over time (dC/dt).

According to this example embodiment, since the microsphere acts as the absolute standard, the impact according to external environmental change, optical system drift, or temperature change may be effectively calibrated, and the measurement stability may be improved by performing spatial averaging using a plurality of microspheres.

### [Embodiment 2: Fixed structure-based refractive index comparison method]

In this example embodiment, a method of measuring the change in refractive index of the culture medium by utilizing a fixed structure formed inside a culture vessel as a reference element is described.

For example, a structure made of a silica material may be formed on the bottom surface of the culture vessel, or the vessel may be manufactured to include a protruding portion in a predetermined shape. The refractive index of the structure may be about 1.46, and may be set to have the higher refractive index than that of the medium.

When the 3D refractive index distribution of an area that includes the structure and the medium is acquired through quantitative phase imaging, the refractive index of the medium may be computed using the refractive index difference at the boundary between the structure and the medium.

If the antibody concentration increases during the progress of the cell culture, the refractive index of the medium may increase and accordingly, the refractive index difference between the structure and the medium may decrease.

For example, when the initial refractive index difference is 0.126, the corresponding difference may decrease to 0.124 according to the increase in the refractive index of the medium.

The system of the present invention may calculate this rate of change to derive the change in refractive index of the medium and to convert the same into the protein concentration. Also, the system may map the relationship between the refractive index and the concentration using prior calibration data.

According to the example embodiment, repeated measurement is possible without addition of a separate microsphere, and reproducibility of measurement conditions is excellent since the structure is integrated into the culture vessel.

### [Embodiment 3: 3D concentration gradient-based single-cell analysis method]

In this example embodiment, a method of analyzing protein secretion at the single-cell level by directly comparing the change in the 3D refractive index distribution over time is described.

When a cell is cultured in a semi-solid matrix or a static culture environment, proteins secreted from the cell may diffuse into surroundings to form a spatial concentration gradient.

By acquiring 3D refractive index distributions at different time points (t1 and t2) and by calculating a differential image between two time points, a refractive index component that increased during the corresponding period may be extracted. Since this change in refractive index corresponds to the change in protein concentration, the concentration may be computed by dividing the amount of increase in refractive index at each location by dn/dc.

By integrating this over the entire space, the total amount of proteins secreted by a specific cell may be calculated, and the secretion rate may be computed by dividing the same by time.

According to the example embodiment, it is possible to quantitatively analyze the secretion characteristics at the single-cell level and to precisely compare the difference in productivity between cells.

### [Embodiment 4: Control group-based measurement calibration method]

In this example embodiment, a method of calibrating a measurement value using a cell group that produces antibodies and a control group is described
Under the same culture conditions, each of a cell group A that produces antibodies and a control group B that does not produce antibodies may be cultured, and the change in refractive index of each of the two culture mediums may be simultaneously or sequentially measured.

The cell group A includes non-specific change due to cellular metabolism along with an increase in refractive index due to antibody secretion, and the control group B reflects only non-specific change.

Therefore, by calculating the difference between the two data, only the pure change in refractive index attributable to antibody secretion may be extracted.

For example, if the total change in refractive index is 0.0010 and 0.0002 is measured from the control group, a difference value of 0.0008 may be used as the actual antibody-attributed change.

According to the example embodiment, various disturbances, such as environmental change, evaporation, and sensor drift, may be effectively eliminated, and high accuracy may be maintained even during long-term measurement.

According to example embodiments, since antibodies may be quantified in a label-free manner that does not use a fluorescent labeling material or an antibody marker, it is possible to minimize additional burden or stress applied to a cell. Therefore, the cell may produce antibodies while maintaining its natural physiological state, and it is possible to reduce the impact of a measurement process on cell growth or productivity.

Also, since measurement may be directly performed within the same vessel during culture, in-line monitoring is possible without a need for separate sample collection. Through this, it is possible to track the continuous change in antibody concentration during the culture process, and to verify dynamic information in real time, such as the change in a production rate, production delay, or cell secretion characteristic. This information may be utilized to control a culture process, and may contribute to optimizing process parameters, such as a nutrient supply timing or a harvesting timing.

Example embodiments may be applied to various culture platforms, such as a multi-well plate and a microfluidic chip, so may measure antibody production for a plurality of cell clones in parallel. Also, since non-destructive continuous measurement is possible at each culture unit, it is possible to significantly reduce a time and a labor required for a process of collecting and individually analyzing samples as in the conventional method. Therefore, production characteristic data on a plurality of candidate cell lines may be quickly secured at an initial development stage, which may contribute to shortening the development period and reducing cost.

In addition, by using physical optical measurement based on the change in refractive index, it is possible to exclude the intervention of human's subjective judgement intervention, and to effectively calibrate errors caused by environmental change through calibration based on a reference element and a control group. Therefore, it is possible to detect even antibodies of low concentration with high precision, and to ensure high reproducibility even during repeated measurement.

Also, example embodiments may be implemented to include a miniatured optical system and data processing unit, and to perform real-time data collection and analysis in conjunction with a culture device. Measured data may be immediately converted into the antibody concentration, and when a set threshold is reached, a process control signal may be generated and linkage to an automated quality management system may be performed.

As a result, example embodiments enable reduction in cell stress, in-line continuous monitoring, high-speed analysis of multiple samples, securing high precision and reproducibility, and easy linkage with process automation, thereby contributing to improving the efficiency and reliability of antibody quantification in a biopharmaceutical production process.

The systems or the apparatuses described herein may be implemented using hardware components, software components, and/or combination of hardware components and software components. For example, the apparatuses and the components described herein may be implemented using one or more general-purpose or special purpose computers, such as, for example, a processor, a controller, an arithmetic logic unit (ALU), a digital signal processor, a microcomputer, a field programmable gate array (FPGA), a programmable logic unit (PLU), a microprocessor, or any other device capable of responding to and executing instructions in a defined manner. The processing device may run an operating system (OS) and one or more software applications that run on the OS. The processing device also may access, store, manipulate, process, and create data in response to execution of the software. For purpose of simplicity, the description of a processing device is used as singular; however, one skilled in the art will be appreciated that a processing device may include multiple processing elements and/or multiple types of processing elements. For example, a processing device may include multiple processors or a processor and a controller. In addition, different processing configurations are possible, such as parallel processors.

The software may include a computer program, a piece of code, an instruction, or some combinations thereof, for independently or collectively instructing or configuring the processing device to operate as desired. Software and/or data may be embodied in any type of machine, component, physical equipment, virtual equipment, computer storage medium or device, to be interpreted by the processing device or to provide an instruction or data to the processing device. The software also may be distributed over network coupled computer systems so that the software is stored and executed in a distributed fashion. The software and data may be stored by one or more computer readable storage media.

The methods according to example embodiments may be configured in a form of program instructions performed through various computer methods and recorded in non-transitory computer-readable media. The media may include, alone or in combination with program instructions, data files and data structures. Here, the media may continuously store computer-executable programs or may transitorily store the same for execution or download. Also, the media may be various types of recording devices or storage devices in a form in which one or a plurality of hardware components are combined. Without being limited to media directly connected to a computer system, the media may be distributed over the network. Examples of the media include ROM, PROM, EPROM, EEPROM, flash memory (e.g., NAND/NOR), SSD, HDD, magnetic tape, optical recording media (CD-ROM, DVD, BD), magneto-optical media, memory card, and USB memory. This storage media refers to non-transitory media, and does not include a transmission signal itself or a pure volatile memory (e.g., RAM). Also, examples of other media may include recording media to storage media managed by an app store that distributes applications or a site, a server, and the like, that supply and distribute other variety of software. Examples of the program instructions include machine language codes as produced by a compiler and advanced language codes executable by a computer using an interpreter.

Although the example embodiments are described with reference to some specific example embodiments and accompanying drawings, it will be apparent to one of ordinary skill in the art that various alterations and modifications in form and details may be made in these example embodiments without departing from the spirit and scope of the claims and their equivalents. For example, suitable results may be achieved if the described techniques are performed in different order, and/or if components in a described system, architecture, device, or circuit are combined in a different manner, and/or replaced or supplemented by other components or their equivalents.

Therefore, other implementations, other example embodiments, and equivalents of the claims are to be construed as being included in the claims.

## Claims

1. A method of quantifying the concentration of secreted proteins in the cell culture medium using optical imaging, the method comprising:
acquiring three-dimensional (3D) data that represents the optical property of the culture medium in which a cell is cultured;
computing the change in refractive index of the culture medium from the 3D data; and
converting the change in refractive index to the concentration of secreted proteins based on the relationship with the protein concentration.

2. The method of claim 1, wherein the computing of the change in refractive index comprises computing the relative or absolute change in refractive index by comparing the refractive index of the culture medium with a reference value.

3. The method of claim 2, wherein the reference value is determined based on at least one of a reference element, initial time point data, control group data, and external calibration data.

4. The method of claim 2, wherein the change in refractive index is calculated based on the difference between a refractive index value as the reference value and a representative refractive index value of a spatially defined area of the culture medium.

5. The method of claim 1, further comprising:
computing the rate of change in concentration of secreted proteins per unit time by comparing 3D data acquired at two different time points.

6. The method of claim 5, wherein the rate of change in concertation per unit time is computed by spatially integrating the local refractive index distribution around a single cell.

7. The method of claim 1, wherein the refractive index of the culture medium is calculated after calibrating the refractive index drift according to the temperature change.

8. The method of claim 7, wherein a culture environment of the culture medium is maintained in the temperature range of ±0.1°C.

9. The method of claim 1, further comprising:
calibrating the change in background refractive index using refractive index data of the culture medium of control cells that do not produce antibodies.

10. The method of claim 1, further comprising:
comparing the concentration of secreted proteins computed for each of a plurality of culture chambers.

11. The method of claim 10, further comprising:
selecting a high-productivity cell line based on the comparison result.

12. The method of claim 1, further comprising:
generating a control signal when the concentration or secretion rate of secreted proteins exceeds a set threshold.

13. The method of claim 1, wherein a culture vessel in which the cell is cultured includes a multi-well plate or a microfluidic device.

14. A secreted protein quantification system for quantifying the concentration of secreted proteins in the cell culture medium using optical imaging, the secreted protein quantification system comprising:
a culture vessel in which a cell is cultured;
an optical imaging device configured to acquire three-dimensional (3D) data that represents the optical property of the culture medium contained in the culture vessel; and
a data processing unit configured to compute the change in refractive index of the culture medium from the 3D data, and to convert the change in refractive index to the concentration of secreted proteins based on the relationship with the protein concentration.

15. The secreted protein quantification system of claim 14, wherein the data processing unit is configured to compute the rate of change in concentration per unit time by comparing the 3D data acquired at different time points, and
the secreted protein quantification system further comprises:
an output unit configured to output the concentration or secretion rate of secreted proteins; and
a process control unit configured to generate a control signal based on the concentration or secretion rate.
